# EUROPÄISCHE PATENTANMELDUNG

(11) **EP 1 074 615 A1**
(43) Veröffentlichungstag der Anmeldung: **07.02.2001**
(21) Anmeldenummer: 00114348.6
(22) Anmeldetag: 05.07.2000
(51) Int. Cl.: C12N 9/64, B01D 15/08, C07K 14/435

(54) **Verfahren zur Reindarstellung der den Blutgerinnungsfaktor VII aktivierenden Protease, ihres Proenzyms oder eines Gemisches beider Proteine mittels Affinitätschromatographie**

(30) Priorität: 06.08.1999 DE 19937218
(71) Anmelder: Aventis Behring GmbH, 35002 Marburg (DE)
(72) Erfinder: Römisch, Jürgen, Dr., 35041 Marburg (DE); Feussner, Annette, 35043 Marburg (DE); Stöhr, Hans-Arnold, 35093 Wetter (DE)

(57) **Zusammenfassung**

Es wird ein Verfahren zur Reindarstellung der den Blutgerinnungsfaktor VII aktivierenden Protease und/oder ihres Proenzyms durch Anwendung eines oder mehrerer affinitätschromatographischer Trennverfahren und/oder einer fraktionierten Fällung beschrieben, bei dem man als affinitätschromatographisches Trennverfahren die Adsorption an
- Kalziumphosphat/Hydroxylapatit,
- einer hydrophoben Matrix,
- einer Chelatmatrix,
- einer Matrix, auf der Heparin oder eine mit Heparin verwandte Substanz wie Heparansulfat oder Dextransulfat immobilisiert ist und/oder
- einer Matrix, die mit einem immobilisierten, gegen das zu isolierende Protein gerichteten, monoklonalen oder polyklonalen Antikörper oder seinen F(ab)- oder F(ab)₂-Fragmenten beschichtet ist, anwendet.

Es werden außerdem eine pharmazeutische Zubereitung und ein Reagenz beschrieben, die die genannte Protease und ihr Proenzym enthalten.

## Beschreibung

Gegenstand der Erfindung ist ein Verfahren zur Reindarstellung der den Blutgerinnungsfaktor VII aktivierenden Protease, ihres Proenzyms oder eines Gemisches beider Proteine sowie von pharmazeutischen Zubereitungen, die die genannten Proteine einzeln oder im Gemisch enthalten.

Aus der deutschen Patentanmeldung 19 903 693.4 ist bereits eine Protease zur Aktivierung des Blutgerinnungsfaktors VII, ein Verfahren zu ihrer Gewinnung, zu ihrem Nachweis und zu ihrer Inaktivierung sowie Arzneizubereitungen bekannt, die diese Protease enthalten. Diese zuerst aus Plasma isolierte Protease kommt dort zusammen mit einer nicht-aktivierten Form vor, die im folgenden als "Proenzym" bezeichnet wird. Die Protease aktiviert den Blutgerinnungsfaktor VII und beschleunigt die Gerinnung, wie durch zahlreiche Experimente gezeigt werden konnte. Bei der weiteren Untersuchung der biologischen Eigenschaften dieses als Serin-Protease identifizierten Proteins stellte sich heraus, dass auch Einketten-Plasminogen-Aktivatoren wie die Prourokinase wirksam aktiviert werden. Außerdem wurde eine Inaktivierung der Faktoren V und VIII in vitro beobachtet. Zusätzlich zu den schon in der deutschen Patentanmeldung 19 903 693.4 beschriebenen sequenzierten Bereichen wurden N-terminale Sequenzierungen von Proteasefragmenten durchgeführt. Folgende Aminosäuresequenzen charakterisieren die FVII-aktivierende Protease: IYGGFKSTAGKHP; LLESLDPDXTPD; EFHEQSFRVEKI; SKFTXAXPXQFK; wobei X nicht identifiziert bedeutet. Die bisher aufgeklärten Sequenzen der genannten Protease zeigen, dass sie zu 100% mit Sequenzen der von Choi-Miura publizierten Protease übereinstimmen (Choi-Miura et al. J. Biochem. 1996; 119: 1157 bis 1165).

Die bisherigen Untersuchungen haben sich vor allem auf die Protease in ihrer aktivierten Form konzentriert. Die im Plasma als Proenzym vorliegende inaktive Form der Protease wurde erst vor kurzer Zeit anhand eines Protein-Bandenmusters in der SDS-PAGE nach Reduktion der Probe aufgefunden. Da bei der Aktivierung der Protease an einer für Serinproteasen typischen Stelle der Primärstruktur eine Spaltung und damit Aktivierung erfolgt, werden bei der Elektrophorese zwei oder mehr Banden sichtbar. Bei Reduktion der über Disulfidbrücken verbundenen Ketten werden die einzelnen Banden entsprechend ihrem niedrigerem Molekulargewicht sichtbar, wobei das Proenzym als große Einzelkette verbleibt. Dies wurde auch in komplexeren Lösungen nach Transfer der Proteine auf Membranen und anschließendes Western Blotting mit geeigneten Antikörpern deutlich.

Aus therapeutischen Gründen besteht nun ein Interesse daran, außer dem Gemisch der beiden genannten Proteine sowohl die Protease in ihrer aktivierten Form als auch das Proenzym zur Verfügung zu haben. Während man die aktivierte Protease zur raschen Aktivierung des Blutgerinnungsfaktors VII oder der Einketten-Plasminogenaktivatoren verwenden kann, um akute Krankheitsbilder zu beeinflussen, ist die Proenzymform der Protease vor allem für die mittel- bis längerfristige Prophylaxe oder Behandlung von angeborenen oder erworbenen Mangelzuständen oder auch zur Erhöhung des Plasmaspiegels über das physiologische Maß hinaus als bevorzugtes Mittel zu wählen. Dabei ist aber zu berücksichtigen, dass die Stabilisierung einer aktivierten Protease schwierig ist, da z.B. eine Eigendegradation stattfinden oder das Molekül aufgrund seiner strukturellen Gegebenheiten instabil sein kann. Bisherige Studien zeigten, dass die den Faktor VII aktivierende Protease nur unter besonderen Umständen in ihrer Proenzymform isoliert und stabilisiert werden kann.

Die bisher vorliegenden Untersuchtungen haben gezeigt, dass die biologischen Aktivitäten dieser Protease durch Kalzium und/oder Heparin oder diesem verwandte Substanzen gesteigert werden können. Diese Eigenschaft wurde schon bisher genutzt, um die Protease an immobilisiertes Heparin zu adsorbieren und eine angereicherte Fraktion zu erhalten. Außerdem ist bereits bekannt, dass die Anionenaustauscher-Chromatographie ebenfalls zur Reinigung der Protease geeignet ist. Die Kombination beider Reinigungsschritte ist geeignet, die Protease in angereicherter Form zu gewinnen. Auch die Verwendung einer Aprotinin-Matrix kann zur Reindarstellung der aktivierten Protease verwendet werden.

Es wurde nun ein Verfahren zur Reindarstellung der den Blutgerinnungsfaktor VII aktivierenden Protease und/oder ihres Proenzyms gefunden, bei dem eines oder mehrere affinitätschromatographische Trennverfahren und/oder eine fraktionierte Fällung eingesetzt werden.

Als affinitätschromatographische Trennverfahren können die Adsorption an
- Kalziumphosphat/Hydroxylapatit,
- einer hydrophoben Matrix,
- einer Chelatmatrix,
- einer Matrix, auf der Heparin oder eine mit Heparin verwandte Substanz wie Heparansulfat oder Dextransulfat immobilisiert ist und/oder eine Matrix, die mit einem immobilisierten, gegen das zu isolierende Protein gerichteten, monoklonalen oder polyklonalen Antikörper oder seinen F(ab)- oder F(ab)₂-Fragmenten beschichtet ist, angewendet werden.

Eine einfache und schnelle Methode zur Anreicherung der Protease und des Proenzyms stellt dabei die Adsorption an Kalziumphosphat/Hydroxylapatit dar. Dabei wird die Lösung, die die Protease und das Proenzym enthält, bei einem pH-Wert zwischen 2,5 und 9,0, vorzugsweise zwischen 2,5 und etwa 7,2 mit Kalziumphosphat versetzt. Nach anschließender Sedimentierung, z.B. durch Zentrifugation oder durch Filtration wird das Sediment, ggf. nach ein- oder mehrfachem Resuspendieren in einer Pufferlösung unter Zugabe von bspw. 0,2 M Natriumcitrat eluiert. Die Protease und das Proenzym befinden sich dann im Eluat.

Auch die Adsorption der Protease an hydrophoben Matrices oder an hydrophoben Liganden, die an ensprechende Matrices gekoppelt sind, kann erfindungsgemäß verwendet werden. Beispiele sind Phenyl- oder Octyl-Sepharosen® oder ein an eine Matrix gekoppeltes Phenytalanin. Die Elution des gebundenen Proteins erfolgt in an sich bekannter Weise mit einer gepufferten Lösung geringer Ionenstärke, die Phenylalanin, Glycerin oder Ethylenglykol enthalten kann.

Da die Protease und das Proenzym eine Wechselwirkung mit Kationen, vor allem mit Kalzium und Magnesiumionen eingehen, was durch eine Steigerung ihrer Aktivität in deren Gegenwart bestätigt wird, bietet sich zu deren Anreicherung aus entsprechenden Lösungen die Chromatographie mittels sogenannter "Chelat-Matrices" an. Chelatverbindungen mit Zink-, Kupfer- oder Nickelionen sind dabei besonders geeignet. Nach dem Waschen der mit der Protease beladenen Matrix kann zur Elution gebundener Proteine auch ein Imidazolpuffer, ggf. mit einem linearen Gradienten, eingesetzt werden.

Das erfindungsgemäße Verfahren lässt sich auch in der Weise durchführen, dass die genannten Proteine durch fraktionierte Fällung, z.B. durch Zusatz von Polyethylenglykol oder Ammoniumsulfat, aus den sie enthaltenden Flüssigkeiten abgetrennt werden. Derartige fraktionierte Fällungen können als alleiniges Trennverfahren eingesetzt werden, jedoch werden die Ausbeute und die Wirksamkeit des erfindungsgemässen Verfahrens weiter verbessert, wenn es in beliebiger Reihenfolge mit anderen, an sich bekannten Reinigungsverfahren kombiniert wird. So kann man durch Beimischung von Polyethylenglykol (vorzugsweise PEG 6000) ab 10% Endkonzentration zu einer den Faktor VII aktivierenden Protease und das Proenzym enthaltenden Lösung im pH-Bereich von 2,5 bis 9,0 eine Fällung der Protease und des Proenzyms durchführen, ohne dass dabei ein Aktivitätsverlust eintritt. Durch eine hiermit erzielbare fraktionierte PEG-Fällung wird eine Trennung von Verunreinigungen möglich. Dies gilt ebenso für die fraktionierte Fällung mittels Ammoniumsulfat, ab etwa 15% Endkonzentration. Die erhaltenen Protease-Präzipitate lassen sich nicht nur ohne Wirkungsverlust lagern, sie sind insbesondere zur Ankonzentrierung der Protease und des Proenzyms geeignet, so dass die Reindarstellung der genannten Proteine in kürzerer Zeit möglich ist und außerdem vermieden wird, dass die Protease aktivierenden Oberflächen ausgesetzt wird, die zu Wirkungsverlusten führen, wie es z.B. bei der Verwendung von Filtern eintritt.

Im allgemeinen ist es jedoch zweckmäßig, alle Verfahrensschritte zur Isolierung der Protease und des entsprechenden Proenzyms aus einer diese Proteine enthaltenen Lösung, wie Plasma, Plasmafraktionen, Gewebeflüssigkeiten oder Zellkulturüberständen der rekombinant oder transgen exprimierten Protease oder deren Mutanten in Gegenwart von Proteinstabilisatoren durchzuführen. Das Gleiche gilt auch für die Lagerung der genannten Proteine und ihre Anwendung in pharmazeutischen Zubereitungen. Besonders zweckmäßig ist es eine Kombination von mehreren Proteinstabilisatoren anzuwenden, wobei die Proteinstabilisatoren ausgewählt werden sollten aus folgenden Substanzgruppen:
- Komplexbildner zweiwertiger Ionen, vorzugsweise EDTA, EGTA oder Citrat, und/oder
- zweiwertigen Ionen, vorzugsweise Kalziumionen, und/oder
- Aminosäuren, vorzugsweise Glutamat, Arginin, Lysin oder Glyzin, und/oder
- Zucker, vorzugsweise Glukose, Arabinose, Mannose oder Mannit, und/oder
- Lösungsvermittlern, vorzugsweise Hydroxyprolin, und/oder
- Detergentien, vorzugsweise Tween® oder Triton®, und/oder
- Alkoholen, vorzugsweise Ethylenglykol oder Polyethylenglykol, und/oder
- Proteinen, vorzugsweise Albumin, Gelatine, Fibronektin, Vitronektin oder ähnlichen Proteinen, und/oder
- Reduktionsmitteln, vorzugsweise Dithiothreitol, Mercaptoethanol oder Cystein, und/oder
- Proteinase-Inhibitoren wie Aprotinin, α-2-Antiplasmin, C1-Esterase-Inhibitor, dem Inter-α-Trypsin-Inhibitor, dem AntithrombinIII/Heparin oder synthetischen Inhibitoren.

Besonders bemerkenswert ist, dass bei den vorstehend beschriebenen Verfahren auch die Proenzymform der Protease in reiner Form erhalten werden kann. Es zeigte sich nämlich, dass unter den genannten sauren Bedingungen aus einer das Proenzym enthaltenden Lösung ein Eluat erhalten werden konnte, das ausschließlich das Proenzym oder wenigstens in sehr stark angereicherten Ausmaß enthielt. Dabei lässt sich die Nativität des so erhaltenen Proenzyms mit Hilfe eines der Aktivitätsteste bestimmen, die in der deutschen Patentanmeldung 199 26 531.3 beschrieben sind, also z.B. durch die photometrische Bestimmung der bei Einwirkung auf chromogene Substrate auftretenden Extinktion oder durch die nach Reduktion der Probe auftretende Einkettenbildung, die durch SDS-PAGE/Western Blotting nachgewiesen werden kann. Das zeigt, dass erfindungsgemäß die Präparation des Proenzyms in schneller und effizienter Weise mit hoher Ausbeute gelingt.

Bei Anwendung der vorstehend genannten Verfahrensschritte kann also sowohl die gereinigte, den Faktor VII aktivierende Protease, ihr Proenzym oder auch ein Gemisch aus der aktivierten Protease und dem Proenzym erhalten werden. Ein besonders empfehlenswerter Weg zur Herstellung einer reinen aktivierten Protease besteht in der chromatographischen Trennung der den Faktor VII aktivierenden Protease von ihrem Proenzym mittels stufenweiser Elution, bei der auf dem Trägermaterial eine Substanz immobilisiert ist, die unterschiedlich starke Bindungen zur Protease einerseits und zum Proenzym andererseits aufweist. Damit können unterschiedliche Eluate gewonnen werden, die entweder nur die aktivierte Protease oder nur das Proenzym enthalten.

Therapeutisch können die genannte aktivierte Protease, das Proenzym oder das Gemisch beider Verbindungen zur Unterstützung der Blutgerinnung bei Blutungsneigung, bei Mangel an Faktoren des endogenen Gerinnungsastes oder als FEIBA (= Factor VIII bypassing activity), aber auch zur endogenen und exogenen Aktivierung von Plasminogenaktivatoren wie der Prourokinase oder des Einketten-tPA verwendet werden. Diese Aktivität kann auch durch Anwendung der genannten Protease zur Prophylaxe oder Therapie von thromboembolischen Erkrankungen in Kombination mit Einketten- oder Zweiketten-Plasminogenaktivatoren oder Antikoagulantien eingesetzt werden. Krankheitsbilder, die mit thrombotischen Komplikationen vergesellschaftet sind, wie Herzinfarkt, Angina pectoris, Schlaganfall oder Beinvenenthrombosen können so erfolgreich behandelt werden.

Ein weiterer Gegenstand der Erfindung ist deshalb eine pharmazeutische Zubereitung, die eine zur Auflösung von fibrinhaltigen Thromben ausreichender Menge der dem Blutgerinnungsfaktor VII aktivierenden Protease und/oder deren Proenzymform enthält. Auch diese Zubereitung kann außerdem Einketten-Plasminogenaktivatoren und/oder Antikoagulantien enthalten. Zweckmässigerweise wird der Zubereitung noch ein Proteinase-Stabilisator oder ein Reduktionsmittel wie Dithiothreitol, Mercaptoethanol oder Cystein zugesetzt, um das Risiko einer Polymerbildung während der Aufarbeitung oder bei der Lagerung zu reduzieren.

Auch bei Wundheilungsprozessen spielen fibrinolytische Prozesse eine Rolle. Dabei kann die genannte Protease und/oder das Proenzym intravenös oder lokal, subkutan, intradermal, intramuskulär oder bei Verletzungen und Wunden als Bestandteil eines Fibrinklebers oder auch topisch oder gebunden an eine geeignete Trägermatrix z.B. in Form eines Vliesses oder eines Pflasters erfolgen, wobei eine Kombination mit Wachstumsfaktoren zweckmässig sein kann. Im allgemeinen kommt eine derartige pharmazeutische Zubereitung in flüssiger oder lyophilisierter Form zur Anwendung, der an sich bekannte Proteinstabilisatoren zugesetzt werden können, also z.B. Komplexbildner, zweiwertige Kationen wie Kalzium, Aminosäuren wie Glutamat, Arginin, Lysin oder Glyzin und/oder Zucker wie Glukose, Arabinose, Mannose oder Mannitol.

Außerdem können die Protease und/oder ihr Proenzym auch zur Beschichtung von in den Organismus zu implantierenden, aus Kunststoffen oder Metallen bestehenden Gegenständen, wie künstlichen Herzklappen, Blutgefässen, aber auch zur Blutentnahme oder künstlichen Ernährung eingeführten Kanülen eingesetzt werden.

Die Erfindung wird durch die folgenden Beispiele erläutert:

### Beispiel 1

### Reindarstellung mittels immobilisierter monoklonaler Antikörper

Monoklonale Antikörper, die gegen die den Faktor VII aktivierende Protease gerichtet sind, wurden an BrCN-Sepharose® gekoppelt. 30 ml dieser mAb-Matrix wurden in eine Säule gefüllt und das Harz mit 50 mM Natriumcitrat, 0,1 M Natriumchlorid (NaCl), 0,1 M Arginin x HCl, pH 6,0, equilibriert.

100 ml Citratplasma wurden über die Säule gepumpt und die Matrix dann mit 50 mM Natriumcitrat, 1 M NaCl, 0,1 M Arginin x HCl, pH 6,0, gewaschen. Danach wurde die Säule mit dem Equilibrierungspuffer erneut gewaschen, woran sich die Elution mit 0,1 M Glyzin, pH 2,5, anschloss. Das Eluat (ca. 30 ml) wurde in einer Vorlage von 3 ml einer 200 mM Natriumcitratlösung, pH 5,5 unter Rühren gesammelt und danach auf einen pH-Wert von 4,5 eingestellt.

Die Eluatlösung wurde zur weiteren Analyse verwendet. Es wurde eine SDS-PAGE mit anschließendem Transfer auf eine PVDF-Membran und eine Detektion der Faktor VII-Aktivator-Bande mit der nicht reduzierten und mit der reduzierten Probe durchgeführt. Aktivitätstests der so gewonnenen Proteine wurden entsprechend dem in der deutschen Patentanmeldung 199 26 531.3 beschriebenen Verfahren, nämlich der Aktivierung von Prourokinase und Faktor VII, mit anschließender Detektion von Urokinase oder dem aktivierten Faktor VII durchgeführt. Die in diesem System getesteten Proteasemengen, bestimmt als Proteaseantigen, entsprechen der erwarteten theoretischen Aktivität, wodurch die Aktivität der isolierten Protease oder des Proenzyms hinsichtlich der biologischen Aktivität gezeigt wurde.

### Beispiel 2

### Anionenaustauscher-Chromatographie

Eine die Proenzymform der Faktor VII-aktivierenden Protease enthaltende Lösung, die noch Verunreinigungen durch andere Proteine enthielt, wurde in einer Pufferlösung aus 20 mM Na-Acetat, 0,1 M Glyzin, pH 4,5, auf eine Mono Q-Sepharose gepumpt und anschließend mit dem o.g. Puffer gewaschen. Die Durchlauf-Fraktion wurde aufgefangen. Die Elution gebundener Proteine erfolgte mit 20 mM Na-Acetat, 2 M NaCl, pH 4,5. Das Eluat wurde in einen Puffer aus 5 mM Na-Citrat, 50 mM NaCl, pH 6,0, verdünnt und in den in Beispiel 1 genannten Testsystemen untersucht. Aliquots wurden bei 4 bis 8°C gelagert bzw. bei - 20°C eingefroren.

Nach Lagerung der Eluatlösung bei 6°C für mehrere Tage wurden die Tests wiederholt, wobei die Verdünnungen der (aufgetauten) Proben jeweils kurz vor dem Test durchgeführt wurden.

SDS-PAGEs/Western Blots bestätigten, dass die Protease in ihrer Proenzymform isoliert worden war. Nach SDS-PAGE und Anfärbung von Proteinen mittels Coomassie Blue waren in der Ausgangslösung (vor Chromatographie) neben der Protease eine Reihe von verunreinigenden Proteinen sichtbar, die ebenfalls in den Durchlauffraktionen zu finden waren. Die Protease stellte sich als eine Bande entsprechend der Proenzymform (also auch nach Reduktion) rein dar. Die Aktivitätstests (siehe Beispiel 1) bestätigten die Nativität der Protease im Sinne der Beibehaltung der biologischen Aktivitäten.

## Patentansprüche

1. Verfahren zur Reindarstellung der den Blutgerinnungsfaktor VII aktivierenden Protease und/oder ihres Proenzyms durch Anwendung eines oder mehrerer affinitätschromatografischer Trennverfahren und/oder einer fraktionierten Fällung, **dadurch gekennzeichnet**, dass man als affinitätschromatografisches Trennverfahren die Adsorption an
- Kalziumphosphat/Hydroxylapatit,
- einer hydrophoben Matrix,
- einer Chelatmatrix,
- einer Matrix, auf der Heparin oder eine mit Heparin verwandte Substanz wie Heparansulfat oder Dextransulfat immobilisiert ist und/oder
- einer Matrix, die mit einem immobilisierten, gegen das zu isolierende Protein gerichteten, monoklonalen oder polyklonalen Antikörper oder seinen F(ab)- oder F(ab)₂-Fragmenten beschichtet ist, anwendet.

2. Verfahren nach Anspruch 1**, dadurch gekennzeichnet**, dass man eines des chromatografischen Trennverfahren oder die fraktionierte Fällung allein oder jedes der vorstehend genannten Verfahren in beliebiger Kombination mit einem anderen der in Anspruch 1 genannten chromatografischen Trennverfahren anwendet.

3. Verfahren nach den Ansprüchen 1 und 2, **dadurch gekennzeichnet**, dass es in Gegenwart von einem oder mehreren Proteinstabilisatoren durchgeführt wird, die ausgewählt werden aus den Gruppen der
- Komplexbildner zweiwertiger Ionen, vorzugsweise EDTA, EGTA oder Citrat, und/oder
- zweiwertigen Ionen, vorzugsweise Kalziumionen, und/oder
- Aminosäuren, vorzugsweise Glutamat, Arginin, Lysin oder Glyzin, und/oder
- Zucker, vorzugsweise Glukose, Arabinose, Mannose oder Mannit, und/oder
- Lösungsvermittlern, vorzugsweise Hydroxyprolin, und/oder
- Detergentien, vorzugsweise Tween® oder Triton®, und/oder
- Alkoholen, vorzugsweise Ethylenglykol oder Polyethylenglykol, und/oder
- Proteinen, vorzugsweise Albumin, Gelatine, Fibronektin, Vitronektin oder ähnlichen Proteinen, und/oder
- Reduktionsmitteln, vorzugsweise Dithiothreitol, Mercaptoethanol oder Cystein, und/oder
- Proteinase-Inhibitoren wie Aprotinin, α-2-Antiplasmin, C1-Esterase-Inhibitor, dem Inter-α-Trypsin-Inhibitor, dem AntithrombinIII/Heparin oder synthetischen Inhibitoren.

4. Verfahren nach den Ansprüchen 1 bis 3, **dadurch gekennzeichnet**, dass man zur affinitätschromatografischen Trennung der den Faktor VII aktivierenden Protease von ihrem Proenzym mittels stufenweiser Elution auf dem Trägermaterial eine Substanz immobilisiert, die unterschiedliche starke Bindungen zur Protease einerseits und zum Proenzym andererseits aufweist, die unterschiedlichen Eluate dann getrennt voneinander auffängt und aus ihnen das jeweilige Protein isoliert.

5. Verfahren nach den Ansprüchen 1 bis 4, **dadurch gekennzeichnet**, dass die fraktionierte Fällung der Protease und/oder ihres Proenzyms aus ihrer Lösung durch Zusatz von
- Polyethylenglykol ab einer Konzentration von mindestens 10 Gew.-% oder
- Ammoniumsulfat ab einer Konzentration von mindestens 15 Gew.-% erfolgt.

6. Pharmazeutische Zubereitung, **dadurch gekennzeichnet**, dass sie die den Blutgerinnungsfaktor VII aktivierende Protease und/oder ihr Proenzym zusammen mit einem oder mehreren Proteinstabilisatoren gemäß dem Anspruch 3 enthält, zur Unterstützung der Blutgerinnung bei Blutungsneigung, bei Mangel an Faktoren des endogenen Gerinnungsweges, als FEIBA, zur Prophylaxe und/oder Therapie von mit thrombotischen Komplikationen vergeselischafteten Krankheitsbildern, bei angeborenen oder erworbenen Mangelzuständen an der Protease oder ihrem Proenzym, zur Unterstützung der Wundheilung allein oder als Bestandteil eines Fibrinklebers, eines Vlieses und in Kombination mit Wachstumsfaktoren zur subkutanen, intramuskulären, intravenösen oder topischen Behandlung.

7. Verwendung einer pharmazeutischen Zubereitung von Anspruch 6 zur Beschichtung von Oberflächen von in den Organismus zu implantierenden, aus Kunststoff oder Metallen bestehenden Gegenständen, wie künstlichen Herzklappen, Blutgefäßen oder zur Blutentnahme oder zur künstlichen Ernährung eingeführten Kanülen.

8. Reagenz enthaltend die den Blutgerinnungsfaktor VII aktivierenden Protease und/oder ihr Proenzym zusammen mit einem oder mehreren Proteinstabilisatoren gemäß Anspruch 3 zur Verwendung in biologischen Testsystemen und zum Antigennachweis.
